(19) Europäisches Patentamt
European Patent Office
Office européen des brevets

(11) **EP 1 584 339 B1**

(12) **EUROPÄISCHE PATENTSCHRIFT**

(45) Veröffentlichungstag und Bekanntmachung des Hinweises auf die Patenterteilung:
**15.07.2009 Patentblatt 2009/29**

(51) Int Cl.:
*A61M 1/16* (2006.01)    *A61M 1/36* (2006.01)

(21) Anmeldenummer: 05013560.7

(22) Anmeldetag: **16.10.1999**

(54) **Verfahren und Vorrichtung zur Überwachung eines Gefäßzugangs**

Method and device for monitoring a blood vessel access

Procédé et dispositif de surveillance d'un accès à un vaisseau sanguin

(84) Benannte Vertragsstaaten:
**DE ES FR GB IT**

(30) Priorität: **20.10.1998 DE 19848235**

(43) Veröffentlichungstag der Anmeldung:
**12.10.2005 Patentblatt 2005/41**

(62) Dokumentnummer(n) der früheren Anmeldung(en) nach Art. 76 EPÜ:
**99120600.4 / 0 995 451**

(73) Patentinhaber: **Fresenius Medical Care Deutschland GmbH**
**61352 Bad Homburg v.d.H. (DE)**

(72) Erfinder: **Kleinekofort, Wolfgang, Dr.**
**65779 Kelkheim (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**EP-A- 0 611 228**        **WO-A-97/10013**
**DE-A- 4 024 434**       **DE-C- 19 734 002**

**Beschreibung**

Die Erfindung betrifft ein Verfahren zur Überwachung eines Gefäßzuganges während einer extrakorporalen Blutbehandlung, insbesondere einer chronischen Blutreinigungstherapie wie Hämodialyse, Hämofiltration und Hämodiafiltration, und eine Vorrichtung zur extrakorporalen Blutbehandlung, insbesondere zur Hämodialyse, Hämofiltration und Hämodiafiltration, mit einer Einrichtung zur Überwachung des Gefäßzuganges.

Bei den bekannten Methoden der chronischen Blutreinigungstherapie wie Hämodialyse, Hämofiltration und Hämodiafiltration wird Blut des Patienten über einen extrakorporalen Kreislauf geleitet. Als Zugang zum Gefäßsystem des Patienten werden arteriovenöse Fisteln, Gefäßimplantate oder auch verschiedene Katheter verwendet. Typische Flüsse innerhalb des Gefäßzugangs liegen im Bereich von 1100 ml/min. Die Konnektion des Patienten mit dem extrakorporalen Kreislauf erfolgt in der Regel über Dialysekanülen, mit denen die Fistel bzw. das Gefäßimplantat punktiert wird.

Falls sich während der Behandlung der Anschluß zwischen extrakorporalem Kreislauf und Gefäßsystem löst bzw. ein Blutleck im extrakorporalen Kreislauf auftritt, kann ein Verbluten des Patienten nur verhindert werden, wenn innerhalb weniger Minuten der extrakorporale Blutfluß gestoppt wird. Daher sind extrakorporale Blutkreisläufe im allgemeinen mit Schutzsystemen ausgestattet, die permanent den arteriellen und venösen Druck ($P_{art.}$ bzw. $P_{ven.}$) innerhalb des Systems sowie den Eintritt von Luft in den extrakorporalen Kreislauf überwachen.

Im Alarmfall wird die Blutbehandlung gestoppt, die venöse Klemme geschlossen sowie ein akustisches und optisches Warnsignal ausgelöst. Das auf der Druckmessung basierende Schutzsystem spricht an, wenn sich der arterielle oder venöse Druck im extrakorporalen Kreislauf um mehr als $\pm 60$ Torr ändert. Hierbei sind die Alarmgrenzen so gewählt, daß eine Lageänderung des Patienten keinen Alarm auslöst.

Falls sich der Anschluß zwischen Patient und Maschine an der arteriellen Verbindung löst, d. h. an derjenigen Kanüle, die den Blutfluß vom Patienten zum extrakorporalen Kreislauf herstellt, spricht das druekbasierende maschinenseitige Schutzsystem schnell an, wie im folgenden begründet wird. Die Dialysekanüle stellt den höchsten Flußwiderstand im extrakorporalen System dar. Wenn Luft über die Kanüle in das arterielle Unterdrucksystem des extrakorporalen Kreislaufs eingesogen wird, sinkt der Flußwiderstand der Kanüle proportional zum Dichteunterschied zwischen Blut und Luft um den Faktor $10^3$. Somit bricht der arterielle Unterdruck im extrakorporalen Kreislauf schlagartig zusammen.

Im Fall, daß sich die venöse Kanüle aus dem Gefäßzugang löst, ist das Ansprechen des druckbasierenden Schutzsystems jedoch nicht immer gewährleistet. Auf der venösen Seite wird das gereinigte Blut dem Patienten mit Überdruck zugeführt, wobei der venöse Überdruck proportional zur Fördermenge der Blutpumpe ist. Ein Eindringen von Luft durch die Kanüle in den extrakorporalen Kreislauf -wie es auf der arteriellen Unterdruckseite der Fall wäre- ist somit ausgeschlossen. Daher ändert sich der Flußwiderstand der venösen Kanüle nicht und der venöse maschinenseitige Druck sinkt lediglich um den Betrag des Drucks im Gefäßzugang des Patienten. Somit ist die venöse Druckänderung im extrakorporalen Kreislauf in der Regel zu klein, um ein Ansprechen des druckbasierenden Schutzsystems auszulösen. Nur im Fall, daß die venöse Kanüle nach dem Herausrutschen aus dem Gefäßzugang deutlich unterhalb der Fistel liegt, bewirkt die zusätzliche hydrostatische Druckdifferenz zwischen venösem Drucksensor und Kanüle einen Maschinenalarm.

Auch im Falle eines Blutlecks im venösen Schlauchsystem kann es vorkommen, daß der resultierende venöse Druckabfall nicht ausreicht, um ein Auslösen des vorhandenen druckbasierenden Schutzsystems zu gewährleisten.

Neben dem obigen Verfahren, bei dem der Druck im arteriellen Zweig des extrakorporalen Kreislaufs überwacht wird, um ein Herausrutschen der arteriellen Kanüle zu erkennen und unabhängig von der Drucküberwachung im arteriellen Zweig der Druck im venösen Zweig des extrakorporalen Kreislaufs überwacht wird, um ein Herausrutschen der venösen Kanüle zu erkennen, sind Überwachungssyteme bekannt, die sich im extrakorporalen Kreislauf fortpflanzende Druckpulse überwachen.

Die WO 97/10013 beschreibt eine Dialysevorrichtung mit einem derartigen Überwachungssystem, das die in der arteriellen Blutleitung durch die Blutpumpe erzeugten Druckpulse in der venösen Blutleitung überwacht.

Der Erfindung liegt die Aufgabe zugrunde, ein Verfahren zur Überwachung eines Gefäßzuganges während einer extrakorporalen Blutbehandlung anzugeben, daß sowohl eine sichere Erkennung des Herausrutschens der venösen Kanüle aus dem Gefäßzugang als auch eine sichere Erkennung eines Blutlecks im venösen Zweig des extrakorporalen Kreislaufs erlaubt und einen geringen apparativen Aufwand erfordert. Diese Aufgabe wird erfindungsgemäß mit den Merkmalen des Patentanspruchs 1 gelöst.

Eine weitere Aufgabe der Erfindung liegt darin, eine Vorrichtung zur extrakorporalen Blutbehandlung mit einer Einrichtung zur Überwachung eines Gefäßzuganges zu schaffen, die sowohl ein Herausrutschen der venösen Kanüle aus dem Gefäßzugang als auch ein Blutleck im venösen Zweig des extrakorporalen Kreislaufs mit hoher Zuverlässigkeit erkennt und nur einen geringen apparativen Aufwand erfordert. Die Lösung dieser Aufgabe erfolgt erfindungsgemäß mit den Merkmalen des Patentanspruchs 6.

Das erfindungsgemäße Verfahren kann als maschinenintegriertes Schutzsystem ausgelegt sein. Hierbei wird von Meßfühlern Gebrauch gemacht, die bereits in den bekannten Blutbehandlungsvorrichtungen vorhanden sind. Somit beschränkt sich die maschinenseitige Änderung zur Implementierung des Schutzssystems lediglich auf eine Modifikation der Maschinensteuerung.

Das erfindungsgemäße Verfahren beruht darauf, daß sowohl der Druck im arteriellen Zweig als auch der Druck im venösen Zweig des extrakorporalen Kreislaufs überwacht wird, um ein Herausrutschen der venösen Kanüle aus dem Gefäßzugang oder ein Blutleck im venösen Zweig des extrakorporalen Kreislaufs zu erkennen. Aus dem Druck im arteriellen und venösen Zweig des extrakorporalen Kreislaufs werden für den Zustand des Gefäßzugangs charakteristische Werte berechnet, die dann zur Erkennung eines fehlerhaften Gefäßzuganges ausgewertet werden.

Mit dem erfindungsgemäßen Verfahren kann nicht nur ein Herausrutschen der venösen Kanüle und ein Blutleck im venösen Zweig des extrakorporalen Kreislaufs sicher erkannt werden, sondern auch ein Herausrutschen der arteriellen Kanüle und ein Blutleck im arteriellen Zweig des extrakorporalen Kreislaufs.

Das erfindungsgemäße Verfahren kann auch mit anderen Verfahren zur Erkennung eines fehlerhaften Gefäßzuganges kombiniert werden. Damit wird die Sicherheit des Überwachungssystems noch weiter erhöht.

Für den Fall, daß der Gefäßzugang fehlerhaft ist, wird vorzugsweise ein akustischer und/oder optischer Alarm gegeben. Darüber hinaus kann der Blutfluß im extrakorporalen Kreislauf unterbrochen werden, um einen Blutverlust zu vermeiden. Eine Unterbrechung des Blutflusses ist bei den bekannten Vorrichtungen zur extrakorporalen Blutbehandlung dadurch möglich, daß die im extrakorporalen Kreislauf angeordnete Blutpumpe angehalten und/oder ein im extrakorporalen Kreislauf angeordnetes Sicherheitsventil, z. B. eine Schlauchklemme, geschlossen wird.

Eine Überwachung des fehlerhaften Gefäßzuganges mit dem erfindungsgemäßen Verfahren kann nicht nur in Vorrichtungen zur Hämodialyse, Hämofiltration und Hämodiafiltration erfolgen, sondern auch in den bekannten Zellseparatoren, in denen das Blut eines Spenders in einem extrakorporalen Kreislauf einer Zentrifugation unterworfen und dabei in seine Bestandteile getrennt wird.

Im folgenden wird das erfindungsgemäße Verfahren zur Überwachung eines Gefäßzuganges sowie eine Vorrichtung zur extrakorporalen Blutbehandlung mit einer Einrichtung zur Überwachung des Gefäßzuganges unter Bezugnahme auf die Zeichnungen anhand eines Ausführungsbeispiels näher erläutert.

Es zeigen:

Fig. 1     eine Tabelle, aus der die Einflüsse ersichtlich sind, die zu einer Änderung des Druckes im arteriellen und venösen Zweig des extrakorporalen Kreislaufs beitragen,

Fig. 2     ein Ausführungsbeispiel einer Vorrichtung zur extrakorporalen Blutbehandlung mit einer Einrichtung zur Überwachung des Gefäßzuganges in vereinfachter schematischer Darstellung,

Fig. 3     einen Flußlaufplan der Überwachungseinrichtung,

Fig. 4     den arteriellen und venösen Druck in Abhängigkeit vom Blutfluß im extrakorporalen Kreislauf,

Fig. 5     die Änderung des arteriellen und venösen Drucks im extrakorporalen Kreislauf bei einer Lageänderung des Patienten,

Fig. 6     die Druckverhältnisse im extrakorporalen Kreislauf beim Herausrutschen der venösen Kanüle aus dem Gefäßzugang und

Fig. 7     die Druckverhältnisse bei einem Blutleck im venösen Zweig des extrakorporalen Kreislaufs.

Während der extrakorporalen Blutbehandlung werden der arterielle und venöse Druck $P_{ven.} + P_{art.}$ im extrakorporalen Kreislauf mit einer Frequenz f gemessen und die Summe $P_S$ aus venösem und arteriellem Druck gebildet (Gleichung 1).

$$P_S := P_{ven.} + P_{art.} \qquad \text{(Gleichung 1)}$$

$P_{art.}$:     Meßwert des arteriellen Drucks im extrakorporalen Kreislauf.
$P_{ven.}$:     Meßwert des venösen Drucks im extrakorporalen Kreislauf.

Zusätzlich wird die Differenz ∆P des venösen und arteriellen Drucks berechnet:

$$\Delta P = P_{ven.} - P_{art.} \qquad \text{(Gleichung 2)}$$

Die Änderung der Summe aus arteriellem und venösem Druck ist gegeben durch:

$$d(P_S) = P_{SN} - P_{SN-1} \qquad \text{(Gleichung 3)}$$

$P_{SN}$: Aktueller Meßwert der Drucksumme.
$P_{SN-1}$: Vorheriger Meßwert der Drucksumme.

Die Änderung der Druckdifferenz beträgt:

$$d(\Delta P) = \Delta P_N - \Delta P_{N-1} \qquad \text{(Gleichung 4)}$$

$\Delta P_N$: Aktueller Meßwert der Druckdifferenz.
$\Delta P_{N-1}$: Vorheriger Meßwert der Druckdifferenz.

Auf einen fehlerhaften Gefäßzugang wird dann geschlossen, wenn für N aufeinanderfolgende Messungen die folgenden Bedingungen erfüllt sind:

a) $\Sigma N\ d(P_s)$ ist negativ und kleiner als ein Schwellenwert $M_1$ (Gleichung 5)
b) $\Sigma N\ d(\Delta P)$ ist negativ und kleiner als ein Schwellenwert $M_2$ (Gleichung 6)

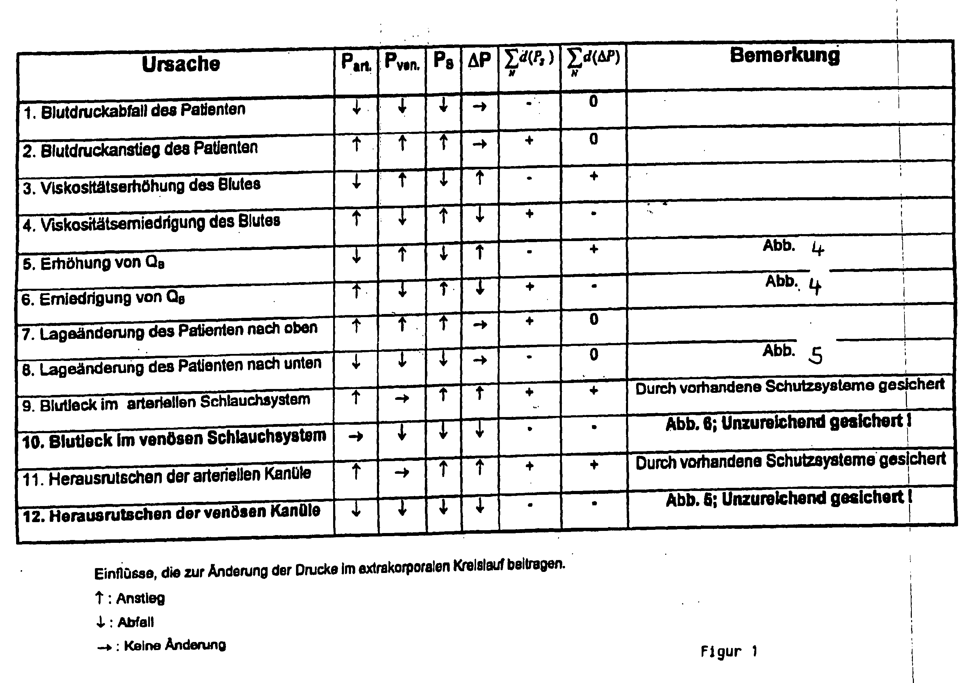

In diesem Fall ist entweder die venöse Kanüle herausgerutscht oder es liegt ein Blutleck im venösen Zweig des extrakorporalen Kreislaufs vor.
Aus der Tabelle (Figur 1) geht hervor, welche Einflüsse zur Änderung des venösen und arteriellen Drucks im extrakorporalen Kreislauf beitragen. Aus der Tabelle ist ersichtlich, daß neben dem Herausrutschen der venösen Kanüle und einem Blutleck im venösen Zweig auch ein Herausrutschen der arteriellen Kanüle und ein Blutleck im arteriellen Zweig des extrakorporalen Kreislaufs erkannt werden können.
Auf ein Herausrutschen der arteriellen Kanüle oder ein Blutleck im arteriellen Zweig des extrakorporalen Kreislaufs kann dann geschlossen werden, wenn die folgenden Bedingungen erfüllt sind:

a) $\Sigma N\ d(P_s)$ ist positiv und größer als ein Schwellenwert $M_3$ (Gleichung 7)
b) $\Sigma N\ d(\Delta P)$ ist positiv und größer als ein Schwellenwert $M_4$ (Gleichung 8)

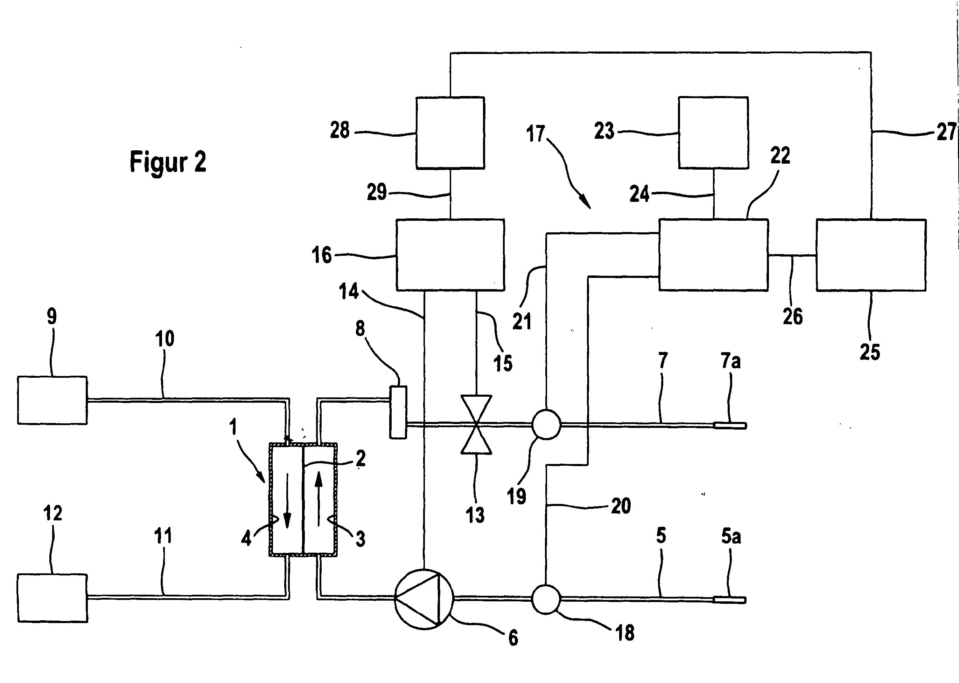

Figur 2 zeigt eine vereinfachte schematische Darstellung einer Dialysevorrichtung mit einer Einrichtung zur Überwachung des Gefäßzuganges.
Die Dialysevorrichtung weist als Blutbehandlungseinrichtung einen Dialysator 1 auf, der durch eine semipermable Membran 2 in eine Blutkammer 3 und eine Dialysierflüssigkeitskammer 4 unterteilt ist. An dem Einlaß der Blutkammer ist eine arterielle Blutleitung 5 angeschlossen, in die eine peristaltische Blutpumpe 6 geschaltet ist. Stromab der Blutkammer 3 führt eine venöse Blutleitung 7 von dem Auslaß der Blutkammer zu dem Patienten. In die venöse Blutleitung 7 ist eine Tropfkammer 8 geschaltet. An den Enden der arteriellen und venösen Blutleitung 5, 7 sind Kanülen 5a, 7a angeschlossen, die in den Patienten gestochen werden. Die arterielle und venöse Blutleitung sind Bestandteil eines als Disposible ausgebildeten Schlauchleitungssystems.
In einer Dialysierflüssigkeitsquelle 9 wird frische Dialysierflüssigkeit bereitgestellt. Von der Dialysierflüssigkeitsquelle 9 führt eine Dialysierflüssigkeitzuführleitung 10 zu dem Eingang der Dialysierflüssigkeitskammer 4 des Dialysators 1, während eine Dialisierflüssigkeitabführleitung 11 von dem Ausgang der Dialysierflüssigkeitskammer zu einem Abfluß 12 führt.
Die Dialysevorrichtung kann noch über weitere Komponenten, z. B. eine Bilanziereinrichtung und eine Ultrafiltrationseinrichtung etc., verfügen, die der besseren Übersichtlichkeit halber nicht dargestellt sind.
Zur Unterbrechung des Blutflusses ist an der venösen Blutleitung 7 stromab der Tropfkammer 8 eine Absperrklemme 13 vorgesehen, die elektromagnetisch betätigt wird. Die arterielle Blutpumpe 6 und die venöse Absperrklemme 13 werden über Steuerleitungen 14, 15 von einer Steuereinheit 16 angesteuert.

Die Einrichtung 17 zur Überwachung des Gefäßzuganges weist einen den Druck in der arteriellen Blutleitung 5 überwachenden arteriellen Drucksensor 18 und einen den Druck in der venösen Blutleitung 7 überwachenden venösen Drucksensor 19 auf. Die Meßwerte der Drucksensoren 18; 19 werden über Datenleitungen 20, 21 an eine Recheneinheit 22 übertragen, die aus den Meßwerten für den Zustand des Gefäßzugangs charakteristische Werte berechnet. In einer Speichereinheit 23, die über eine Datenleitung 24 mit der Recheneinheit 22 verbunden ist, werden die bei der Berechnung anfallenden Zwischenergebnisse abgelegt. Zur Auswertung der für den Gefäßzugang charakteristischen Werte weist die Überwachungseinheit eine Auswerteinheit 25 auf, die über eine Datenleitung 26 mit der Recheneinheit verbunden ist. Die Auswerteinheit 25 ist über eine Steuerleitung 27 an eine Alarmeinheit 28 angeschlossen, die über eine Steuerleitung 29 mit der Steuereinheit 16 verbunden ist.

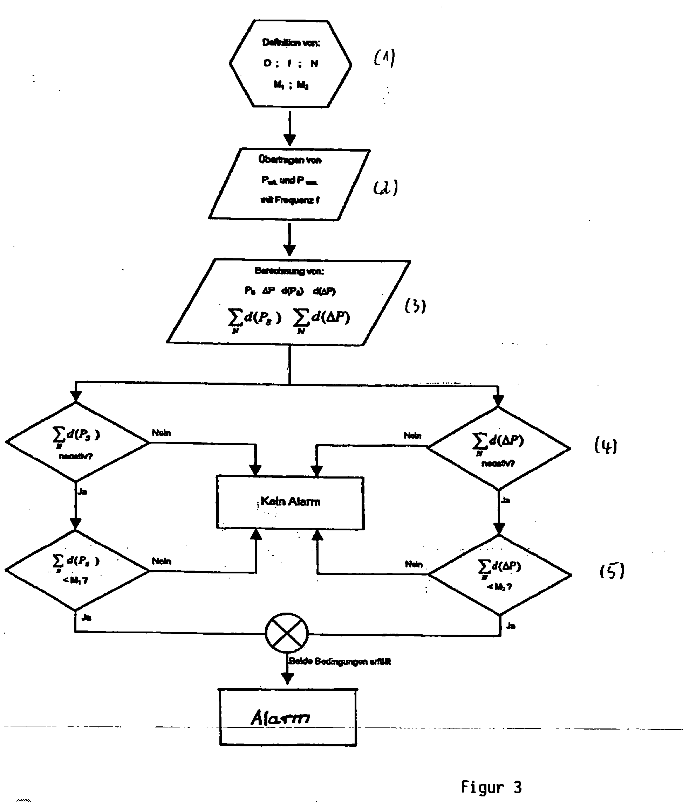

Nachfolgend wird die Funktionsweise der Überwachungseinrichtung 17 unter Bezugnahme auf Figur 3 im einzelnen beschrieben.

Zunächst werden die für die Auswertung heranzuziehende Anzahl N aufeinanderfolgender Meßwerte, die Meßfrequenz f sowie die Schwellwerte $M_1$ und $M_2$ festgelegt. Diese Werte können in der Speichereinheit 23 abgelegt sein oder auch vom Benutzer vorgegeben werden (Schritt 1).

Durch die Auswertung von N aufeinanderfolgenden Meßwerten wird die Störanfälligkeit des Schutzsystems, z. B. bei kurzzeitigen artifiziellen Druckschwankungen reduziert. Der Wert für N ist ganzzahlig und abhängig von der Frequenz f, mit welcher die Druckwerte ermittelt werden. Er kann der jeweiligen Dämpfung D der Drucksensoren angepaßt werden. Für N gilt fogende Randbedingung:

$$N > f \cdot D \qquad \text{(Gleichung 9)}$$

Bei einer typischen Meßfrequenz f von z. B. 1/3 Hz und einer Dämpfung von ca. 18 s sollte N folglich > 6 sein. Hierdurch ist gewährleistet, daß der Druckabfall vollständig erkannt wird.

Die negativen Schwellenwerte $M_1$ und $M_2$ kennzeichnen die Empfindlichkeit des Schutzsystems. Generell gilt: Je kleiner $M_1$ und $M_2$, desto höher liegt die Schwelle, bei welcher ein Maschinenalarm ausgelöst wird. Um ein Ansprechen des Schutzsystems zu gewährleisten, müssen die Werte für $M_1$ und $M_2$ jedoch größer als der negative Wert des venösen Drucks im Gefäßzugang sein.

Der arterielle Fisteldruck sinkt nach dem Herausrutschen der venösen Kanüle aus dem Gefäßzugang durch die Blutung an der venösen Punktionsstelle geringfügig ab. Dieser Effekt kann durch die geeignete Wahl von $M_2$ berücksichtigt werden, wobei die Bedingung $M_1 < M_2$ gelten muß. Geeignete Werte sind z. B. $M_1 = -15$ Torr und $M_2 = -10$ Torr.

Die Ansprechzeit des Schutzsystems $T$ ist gegeben durch:

$$\tau = \frac{N}{f} \qquad \text{(Gleichung 10)}$$

Während der Dialysebehandlung werden mittels der Druckmeßsensoren 18, 19 der arterielle und venöse Druck $P_{art.}$, $P_{ven.}$ mit der Meßfrequenz f in N aufeinanderfolgenden Messungen erfaßt (Schritt 2).

Die Recheneinheit 22 berechnet nach jeder Messung gemäß Gleichung 1 die Summe $P_s$ des venösen und arteriellen Drucks und gemäß Gleichung 2 die Differenz $\Delta P$ des venösen und arteriellen Drucks. Diese Werte werden in der Speichereinheit 23 abgelegt. Nach Gleichung 3 berechnet die Recheneinheit 22 die Differenz $d(P_s)$ zwischen der Summe $P_{SN}$ des venösen und arteriellen Drucks einer nachfolgenden Messung und der Summe $P_{SN-1}$ des venösen und arteriellen Drucks einer vorhergehenden Messung. Die Differenz $d(\Delta P)$ zwischen der Differenz $d(\Delta P)$ des arteriellen und venösen Drucks einer vorhergehenden Messung $\Delta P_N$ und der Differenz $\Delta P_{N-1}$ des arteriellen und venösen Drucks einer nachfolgenden Messung berechnet die Recheneinheit 22 nach Gleichung 4. Auch diese Meßwerte werden in der Speichereinheit 23 abgelegt. Die Summenänderungen $d(P_S)$ der N aufeinanderfolgenden Messungen werden dann zur Berechnung eines ersten den Zustand des Gefäßzuganges charakteristischen Wertes $W_1$ in der Recheneinheit 22 nach Gleichung 5 addiert. Zur Berechnung eines den Gefäßzustand charakteristischen zweiten Wertes $W_2$ werden in der Recheneinheit 22 die Differenzänderungen $d(\Delta P)$, nach Gleichung 6 addiert (Schritt 3).

Die Auswerteinheit 25 überprüft das Vorzeichen der beiden charakteristischen Werte $W_1$ und $W_2$. Sind der erste oder der zweite charakteristische Wert $W_1$, $W_2$ negativ, so erfolgt kein Alarm (Schritt 4). Wenn jedoch sowohl der erste als auch der zweite charakteristische Wert $W_1$, $W_2$ negativ sind, werden die charakteristischen Werte in der Auswerteinheit 25 mit dem ersten und zweiten Schwellwert M1, M2 verglichen (Schritt 5).

Für den Fall, daß sowohl $W_1$ kleiner als $M_1$ als auch $W_2$ kleiner $M_2$, gibt die Auswerteinheit 25 ein Alarmsignal an die Alarmeinheit 28 ab. Die Alarmeinheit 28 erzeugt einen akustischen und/oder optischen Alarm und steuert die Steuereinheit 16 an, die wiederum die Blutpumpe 6 anhält und das Absperrorgan 13 schließt. Damit ist sichergestellt, daß in dem Fall, daß die venöse Kanüle 7a herausgerutscht ist oder ein Leck in der venösen Blutleitung 7 vorliegt der Patient nicht gefährdet ist.

In analoger Weise kann nach Gleichungen 7 und 8 auf einen fehlerhaften Gefäßzustand aber auch dann geschlossen werden, wenn sowohl der erste charakteristische Wert $W_1$ als auch der zweite charakteristische Wert $W_2$ positiv sind und der erste charakteristische Wert $W_1$ größer als ein vorgegebener Schwellwert $M_3$ und der zweite charakteristische Wert $W_2$ größer als ein vorgegebener Schwellwert $M_4$ ist. In diesem Fall ist die arterielle Kanüle herausgerutscht oder es liegt ein Blutleck im arteriellen Zweig des extrakorporalen Kreislaufs vor.

Die Recheneinheit 22 mit der Speichereinheit 23 und die Auswerteinheit 25 können Bestandteil des Mikrocomputers sein, der in den bekannten Dialysevorrichtungen ohnehin vorhanden ist.

Sofern die Dialysevorrichtung über eine Ultrafiltrationseinrichtung verfügt, wird die Überwachungseinrichtung innnerhalb der ersten ein bis zwei Minuten nach dem Ein- bzw. Ausschalten der Ultrafiltrationseinrichtung deaktiviert, um einen Fehlalarm zu vermeiden.

Nachfolgend werden die charakteristischen Grundlagen erläutert, auf denen das erfindungsgemäße Verfahren zur Überwachung des Gefäßzugangs beruht.

Die im extrakorporalen Kreislauf gemessenen Drucke $P_{art.}$ und $P_{ven.}$ setzen sich aus dem dynamischen Druck im Extrakorporalsystem, der durch den Fluß der Blutpumpe erzeugt wird, und dem dynamischen Druck im Gefäßzugang des Patienten zusammen.

Hierbei ist der dynamische Druck im Extrakorporalsystem eine Funktion des extrakorporalen Blutflusses sowie der Summe der Flußwiderstände im extrakorporalen Kreislauf. Da sich arterieller und venöser Flußwiderstand aufgrund der unterschiedlichen Geometrie der durchströmten Komponenten unterscheiden, ist die Drucksumme $P_s$ ebenfalls eine Funktion des Blutflusses.

In der Regel wird bei den bekannten Blutreinigungsverfahren die Förderrate der Blutpumpe $Q_B$ auf einen festen Wert eingestellt. Somit ist die Summe der Flußwiderstände im extrakorporalen Kreislauf bei konstanter Viskosität des Blutes ebenfalls konstant. Eine Erhöhung der Viskosität, z. B. durch Ultrafiltration, führt langfristig zur Erniedrigung des arteriellen Unterdrucks und zur Erhöhung des venösen Überdrucks. Folglich ist das Druckverhalten bei stetiger Viskositätserhöhung identisch mit einer geringen Erhöhung des Blutflusses.

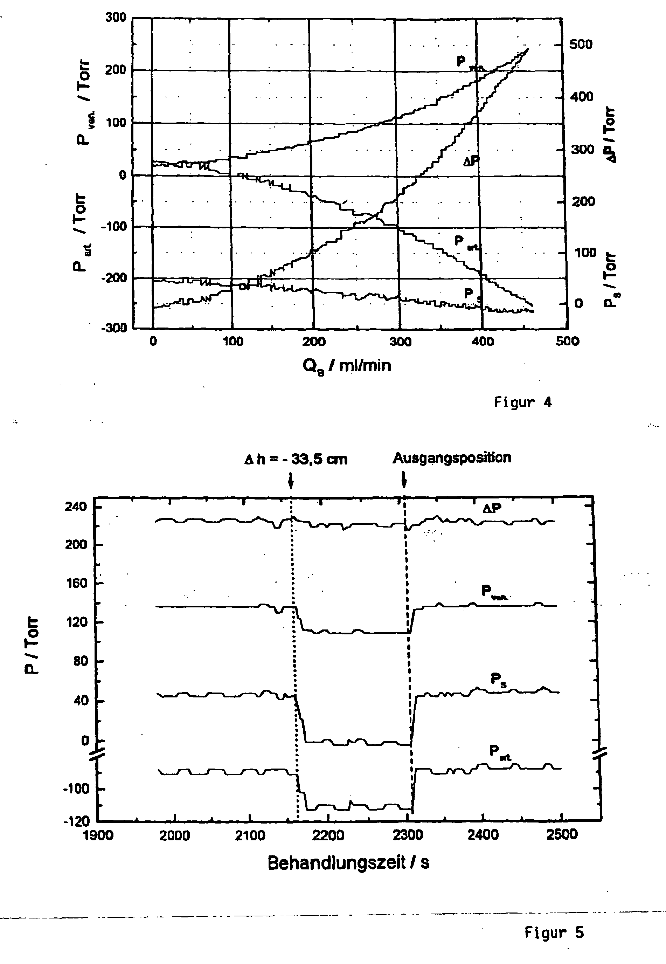

Figur 4 zeigt die extrakorporalen Drucke in Abhängigkeit vom Blutfluß $Q_B$ bei konstanter Viskosität (Fistelfluß $Q_F$ = 1255 ml/min, arterieller Fisteldruck $P_{F\,art.}$ = 27 Torr, venöser Fisteldruck $P_{Fven.}$ = 17 Torr).

Die obige und alle folgenden Messungen wurden während einer simulierten Dialysebehandlung durchgeführt. Als Gefäßzugang wurde ein Schlauchsegment mit einem Innendurchmesser von 8 mm verwendet, welches mit einer Zahnradpumpe verbunden war. Zur Messung des Flusses innerhalb des Gefäßzugangs wurde ein Doppler-Flußmesser verwendet. Die arteriellen und venösen Drucke innerhalb des Gefäßzugangs ($P_{Fistel}$) wurden mit zwei Druckmanometern kontrolliert und konnten über Schlauchklemmen eingestellt werden. Bei allen Messungen wurde Wasser verwendet (T=37°C). Zur Gefäßpunktion wurden Kanülen verwendet, die über zwei konventionelle Schlauchsysteme mit dem Dialysegerät verbunden waren.

Der dynamische Druck im Gefäßzugang des Patienten, im folgenden Fisteldruck genannt, ist ebenfalls eine Funktion der Blutviskosität, des systemischen Blutdrucks sowie der systemischen vaskulären Flußwiderstände. Der Fisteldruck ist somit ein patientenspezifischer Parameter und hängt zusätzlich ab von der Art des Gefäßzugangs, der Viskosität des Blutes sowie vom Gefäßsystem, das den Gefäßzugang mit Blut versorgt. Analog zum dynamischen Druck im Extrakorporalsystem führt eine Änderung des Fisteldrucks, z. B. durch Blutdruckschwankung, Viskositätserhöhung oder Lageänderung des Patienten zur Änderung sowohl des arteriellen als auch des venösen Druckwertes.

Figur 5 zeigt das Verhalten der extrakorporalen Drucke bei konstantem effektiven Blutfluß $Q_B$, wenn sich die Lage des Patienten relativ zu den extrakorporalen Drucksensoren um Δh=-33,5 cm ändert ($Q_B$= 300 ml/min, $Q_F$=1252 ml/min; Druck im Gefäßzugang: $P_{F\,art.}$ = 27 Torr, $P_{F\,ven.}$ = 17 Torr). Dies ist z. B. der Fall, wenn sich der Patient hinlegt bzw. seine Liege herunterfährt. Hierbei reduzieren sich die arteriellen und venösen Druckwerte um den Betrag der hydrostatischen Druckdifferenz (ca. 0,78 Torr pro cm Höhendifferenz zwischen Drucksensor und Fistel). Da sich bei einer Lageänderung des Patienten der arterielle und venöse Druck im extrakorporalen Kreislauf um den gleichen Wert ändert, bleibt die Druckdifferenz ΔP konstant. Hingegen sinkt die Summe der Drucke $P_s$ um den doppelten Betrag der hydrostatischen Druckdifferenz.

Dieses Verhalten zeigt sich (allerdings in abgeschächter Form) auch bei einem Blutdruckabfall während der Behandlung. In diesem Falle sinken ebenfalls der arterielle und venöse Druck im extrakorporalen Kreislauf.

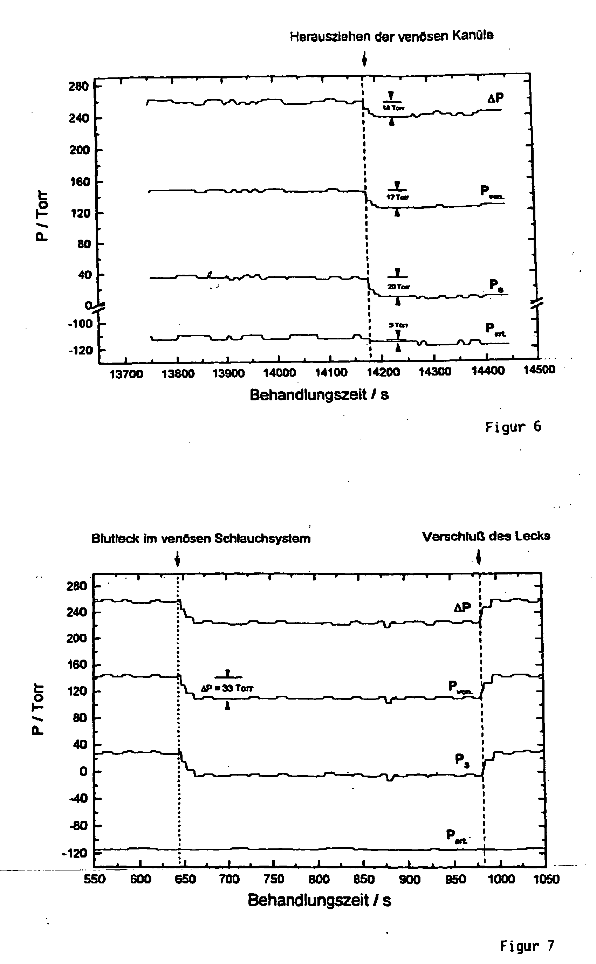

Figur 6 zeigt die Druckverhältnisse beim Herausrutschen der venösen Dialysekanüle aus der Fistel des Patienten ($Q_B$=300 ml/min, $Q_F$=1254 ml/min, $P_{F\,art.}$=27 Torr, $P_{F\,ven.}$ =17 Torr). Der Druck am venösen Sensor des extrakorporalen Kreislaufs ($P_{ven.}$) verringert sich innerhalb von ca 15-20 Sekunden um den Betrag des venösen Fisteldrucks. Die Verzögerungszeit ist durch die elektronische Dämpfung der extrakorporalen Drucksignale bedingt. Der arterielle Druckwert ($P_{art.}$) reduziert sich innerhalb der ersten Minuten nur geringfügig, wie im folgenden begründet wird.

Der Blutverlust, welcher durch die offene venöse Punktionsstelle bedingt ist und zum Absinken des Fisteldrucks führt, kann über den Energieerhaltungssatz grob abgeschätzt werden:

$$P \cdot V = \frac{1}{2} M v^2 \qquad (Gleichung\,11)$$

mit folgenden Abkürzungen:

P: Druck
V: Volumen
M: Masse
v: Geschwindigkeit

Mit $V = M/\rho$ folgt für die Geschwindigkeit des Blutstrahls aus der offenen Punktionsstelle:

$$v = \sqrt{\frac{2P}{\rho}} \qquad (Gleichung\,12)$$

$\rho$: Dichte

Bei einem venösen Fisteldruck von 17 Torr (=22,6 mbar) und einer Blutdichte von 1,0506 g/cm³ (37°C) beträgt die Strahlgeschwindigkeit ca. 2,1 m/s. Unter der Annahme einer gleichbleibenden Öffnung der Punktionsstelle ist der Volumenstrom bei zylindrischem Strahlprofil gegeben durch:

$$Q = \pi \cdot r^2 v \qquad (Gleichung\,13)$$

Bei einem typischen Kanülendurchmesser von 1,6 ergibt sich ein Wert von ca. 250 ml/min. (=1/5 des Fistelflusses). In vivo schwillt die offene Punktionsstelle an, wodurch der effektive Lochdurchmesser kleiner als der Kanülendurchmesser sein wird. Somit ist der angegebene Wert als Obergrenze anzusehen.

Da der Druck und Fluß proportional sind, reduziert sich der venöse Fisteldruck folglich um 17/5 = 3,4 Torr (Gesetz von Hagen-Pbiseuille). Durch den verminderten venösen Fisteldruck sinkt der arterielle extrakorporale Druck um den gleichen Wert. Da die arterielle Druckänderung jedoch immer kleiner als die venöse Druckänderung ist, verringern sich sowohl $P_s$ als auch $\Delta P$.

Figur 7 zeigt die Druckverhältnisse bei einem Blutleck im venösen Schlauchsystem. Hierzu wurde der venöse Schlauch unterhalb der Tropfkammer mit einer Injektionskanüle punktiert ($Q_B$=300 ml/min, $Q_F$=1250ml/min, $P_{F\,art.}$ = 27 Torr, $P_{F\,ven.}$ = 17 Torr, Leckrate 50 ml/min). Der Druck am venösen Sensor verringert sich bei einer Leckrate von 50 ml/min innerhalb von ca. 15-20 s um rund 33 Torr. Der arterielle Druck hingegen ändert sich nicht merklich. Analog zu Figur 6 sinken sowohl $\Delta P$ als auch $P_S$, folglich sind die Summenänderungen nach Gleichung 6 und 7 in beiden Fällen negativ.

**Patentansprüche**

1. Verfahren zur Überwachung eines Gefäßzuganges während einer extrakorporalen Blutbehandlung, bei der Blut aus dem Gefäßzugang über einen arteriellen Zweig eines extrakorporalen Blutkreislaufs in eine Blutbehandlungseinrichtung strömt und aus der Blutbehandlungseinrichtung über einen venösen Zweig des extrakorporalen Kreislaufs zurück in den Gefäßzugang strömt, wobei der Druck im arteriellen und im venösen Zweig des extrakorporalen Kreislaufs überwacht wird, und aus dem Druck im arteriellen und venösen Zweig des extrakorporalen Kreislaufs für den Zustand des Gefäßzugangs charakteristische Werte berechnet werden, die zur Erkennung eines fehlerhaften Gefäßzuganges ausgewertet werden, **dadurch gekennzeichnet, daß** der Druck im venösen und arteriellen Zweig des extrakorporalen Kreislaufs in aufeinanderfolgenden Messungen erfaßt wird, wobei die Summe und die Differenz des venösen und arteriellen Drucks im extrakorporalen Kreislauf bestimmt werden, und daß aus der Summe und Differenz des venösen und arteriellen Drucks die für den Zustand des Gefäßzuganges charakteristischen Werte berechnet werden.

2. Verfahren nach Anspruch 1, **dadurch gekennzeichnet, daß** zur Berechnung eines ersten den Zustand des Gefäßzuganges charakteristischen Wertes die Differenz zwischen der Summe des venösen und arteriellen Drucks einer nachfolgenden Messung und der Summe des venösen und arteriellen Drucks einer vorhergehenden Messung ermittelt und diese Druckdifferenzen aufeinanderfolgender Messungen addiert werden und zur Berechnung eines zweiten den Zustand des Gefäßzuganges charakteristischen Wertes die Differenz zwischen der Differenz des venösen und arteriellen Drucks einer nachfolgenden Messung und der Differenz des venösen und arteriellen Drucks einer vorhergehenden Messung ermittelt und diese Druckdifferenzen aufeinanderfolgender Messungen fortlaufend addiert werden.

3. Verfahren nach Anspruch 2, **dadurch gekennzeichnet, daß** geprüft wird, ob der erste und zweite charakteristische Wert positiv oder negativ sind, und daß der erste und zweite charakteristische Wert mit einem ersten und zweiten Schwellwert verglichen wird, wobei auf einen fehlerhaften Gefäßzugang dann geschlossen wird, wenn sowohl der erste und zweite charakteristische Wert negativ als auch der erste charakteristische Wert kleiner als der erste Schwellwert und der zweite charakteristische Wert kleiner als der zweite Schwellwert sind.

4. Verfahren nach einem der Ansprüche 1 bis 3, **dadurch gekennzeichnet, daß** bei Feststellung eines fehlerhaften Gefäßzuganges ein Alarm gegeben wird.

5. Verfahren nach einem der Ansprüche 1 bis 4, **dadurch gekennzeichnet, daß** bei Feststellung eines fehlerhaften Gefäßzuganges der Blutfluß im extrakorporalen Kreislauf unterbrochen wird.

6. Vorrichtung zur extrakorporalen Blutbehandlung mit
einer arteriellen Blutleitung (5) eines extrakorporalen Blutkreislaufs, die an einem Ende mit dem Einlaß einer Blutbehandlungseinrichtung (1) verbunden ist und an dem anderen Ende mit einem arteriellen Anschluß (5a) für einen Gefäßzugang versehen ist,
einer venösen Blutleitung (7) des extrakorporalen Blutkreislaufs, die an einem Ende mit dem Auslaß der Blutbehandlungseinrichtung (1) verbunden ist und an dem anderen Ende mit einem venösen Anschluß (7a) für den Gefäßzugang versehen ist, und
einer Einrichtung (17) zur Überwachung des Gefäßzuganges mit einem den Druck $P_{art.}$ in der arteriellen Blutleitung überwachenden arteriellen Drucksensor (18) und einen den Druck $P_{ven.}$ in der venösen Blutleitung überwachenden venösen Drucksensor (19),
wobei
die Einrichtung zur Überwachung des Gefäßzuganges aufweist:

eine Recheneinheit (22) zum Berechnen von für den Zustand des Gefäßzugangs charakteristischen Werten $W_1$, $W_2$ aus dem Druck im arteriellen und venösen Zweig des extrakorporalen Kreislaufs und
eine Auswerteinheit (25) zur Auswertung der charakteristischen Werte, um einen fehlerhaften Gefäßzugang zu erkennen,
**dadurch gekennzeichnet, daß** die Recheneinheit (22) Mittel zum Berechnen der Summe und der Differenz des in aufeinanderfolgenden Messungen erfaßten venösen und arteriellen Drucks im extrakorporalen Kreislauf aufweist.

7. Vorrichtung nach Anspruch 6, **dadurch gekennzeichnet, daß** die Einrichtung (17) zur Überwachung des Gefaßzuganges Mittel (23) zum Speichern der in aufeinanderfolgenden Messungen ermittelten Summen und Differenzen des venösen und arteriellen Drucks aufweist und daß die Recheneinheit (22) zur Berechnung eines ersten den Zustand des Gefäßzuganges charakteristischen Wertes $W_1$ Mittel zum Bilden der Differenz zwischen der Summe des venösen und arteriellen Drucks einer nachfolgenden Messung und der Summe des venösen und arteriellen Drucks einer vorhergehenden Messung und Mittel zum Addieren dieser Druckdifferenzen aufeinanderfolgender Messungen aufweist und zur Berechnung eines zweiten den Zustand des Gefäßzuganges charakteristischen Wertes $W_2$ Mittel zum Bilden der Differenz zwischen der Differenz des venösen und arteriellen Drucks einer nachfolgenden Messung und der Differenz des venösen und arteriellen Drucks einer vorhergehenden Messung und Mittel zum Addieren dieser Druckdifferenzen aufeinanderfolgender Messungen aufweist

8. Vorrichtung nach Anspruch 7 **dadurch gekennzeichnet, daß** die Auswerteinheit (25) Mittel zum Bestimmen des Vorzeichens des ersten und zweiten charakteristischen Wertes $W_1$, $W_2$ und Mittel zum Vergleichen des ersten charakteristischen Wertes mit einem ersten Schwellwert $M_1$ und Mittel zum Vergleichen des zweiten charakteristischen Wertes $W_2$ mit einem zweiten Schwellwert $M_2$ aufweist.

9. Vorrichtung nach einem der Ansprüche 6 bis 8, **dadurch gekennzeichnet, daß** ein Alarmgeber (28) vorgesehen ist, der bei Feststellung eines fehlerhaften Gefäßzuganges einen Alarm auslöst.

10. Vorrichtung nach einem der Ansprüche 6 bis 9, **dadurch gekennzeichnet, daß** Mittel (16, 28) zum Unterbrechen des Blutflusses im extrakorporalen Blutkreislauf bei der Feststellung eines fehlerhaften Gefäßzuganges vorgesehen sind.

**Claims**

1. Method of monitoring a blood vessel access during extra-corporeal blood treatment in which blood flows from the blood vessel access via an arterial segment of an extra-corporeal blood circuit to a blood treating arrangement and flows from the blood treating arrangement via a venous segment of the extra-corporeal circuit back to the blood vessel access, the pressures in the arterial and venous segments of the extra-corporeal circuit being monitored, and values characteristic of the state of the blood vessel access being calculated from the pressures in the arterial and venous segments of the extra-corporeal circuit, which values are analysed to allow a faulty blood vessel access to be detected, **characterised in that** the pressures in the venous and arterial segments of the extra-corporeal circuit are sensed in successive measurements, the sum and the difference of the venous and arterial pressures in the extra-corporeal circuit being determined, and **in that** values characteristic of the state of the blood vessel access are calculated from the sum and difference of the venous and arterial pressures.

2. Method according to claim 1, **characterised in that**, to allow a first value characteristic of the state of the blood vessel access to be calculated, the difference between the sum of the venous and arterial pressures in a succeeding measurement and the sum of the venous and arterial pressures in a preceding measurement is determined and these differences in pressure in successive measurements are added up and, to allow a second value characteristic of the state of the blood vessel access to be calculated, the difference between the difference in the venous and arterial pressures in a succeeding measurement and the difference in the venous and arterial pressures in a preceding measurement is determined and these differences in pressure in successive measurements are continuously added up.

3. Method according to claim 2, **characterised in that** a check is made to see whether the first and second characteristic values are positive or negative and **in that** the first and second characteristic values are compared with first and second threshold values, it being concluded that there is a faulty blood vessel access when both the first and second characteristic values are negative and the first characteristic value is also lower than the first threshold value and the second characteristic value is also lower than the second threshold value.

4. Method according to one of claims 1 to 3, **characterised in that** an alarm is given if there is found to be a faulty blood vessel access.

5. Method according to one of claims 1 to 4, **characterised in that** the flow of blood in the extra-corporeal circuit is interrupted if there is found to be a faulty blood vessel access.

6. Apparatus for extra-corporeal blood treatment having
   an arterial blood line (5) belonging to an extra-corporeal blood circuit, which arterial blood line (5) is connected to the inlet of a blood treating arrangement (1) at one end and to an arterial connection (5a) for a blood vessel access at the other end,
   a venous blood line (7) belonging to the extra-corporeal blood circuit, which venous blood line (7) is connected to the outlet of the blood treating arrangement (1) at one end and to a venous connection (7a) for the blood vessel access at the other end, and
   an arrangement (17) for monitoring the blood vessel access which has an arterial pressure sensor (18) monitoring the pressure $P_{art.}$ in the arterial blood line and a venous pressure sensor (19) monitoring the pressure $P_{ven.}$ in the venous blood line,
   the arrangement for monitoring the blood vessel access having:

a calculating unit (22) for calculating values $W_1$, $W_2$ characteristic of the state of the blood vessel access from the pressures in the arterial and venous segments of the extra-corporeal circuit, and
an analysing unit (25) for analysing the characteristic values to allow a faulty blood vessel access to be detected, **characterised in that** the calculating unit (22) has means for calculating the sum and difference of the venous and arterial pressures in the extra-corporeal circuit which are sensed in successive measurements.

7. Apparatus according to claim 6, **characterised in that** the arrangement (17) for monitoring the blood vessel access has means (23) for storing the sums and differences of the venous and arterial pressures which are determined in successive measurements, and **in that**, to allow a first value $W_1$ characteristic of the state of the blood vessel access to be calculated, the calculating unit (22) has means for forming the difference between the sum of the venous and arterial pressures in a succeeding measurement and the sum of the venous and arterial pressures in a preceding measurement and means for adding up these differences in pressure in successive measurements and, to allow a second value $W_2$ characteristic of the state of the blood vessel access to be calculated, the calculating unit (22) has means for forming the difference between the difference in the venous and arterial pressures in a succeeding measurement and the difference in the venous and arterial pressures in a preceding measurement and means for adding up these differences in pressure in successive measurements.

8. Apparatus according to claim 7, **characterised in that** the analysing unit (25) has means for determining the sign of the first and second characteristic values $W_1$, $W_2$ and means for comparing the first characteristic value $W_1$ with a first threshold value $M_1$ and means for comparing the second characteristic value $W_2$ with a second threshold value $M_2$.

9. Apparatus according to one of claims 6 to 8, **characterised in that** an alarm initiator (28) is provided which triggers an alarm if there is found to be a faulty blood vessel access.

10. Apparatus according to one of claims 6 to 9, **characterised in that** means (16, 28) are provided to interrupt the flow of blood in the extra-corporeal blood circuit if there is found to be a faulty blood vessel access.

**Revendications**

1. Procédé de surveillance d'un accès vasculaire pendant un traitement extracorporel du sang, dans lequel le sang s'écoule de l'accès vasculaire en passant par une branche artérielle d'un circuit extracorporel du sang dans un dispositif de traitement du sang et du dispositif de traitement du sang en passant par une branche veineuse du circuit extracorporel de nouveau dans l'accès vasculaire, dans lequel la pression dans les branches artérielle et veineuse du circuit extracorporel est surveillée, et à partir de la pression dans les branches artérielle et veineuse du circuit extracorporel, des valeurs caractéristiques pour l'état de l'accès vasculaire sont calculées, qui sont évaluées pour identifier un accès vasculaire défectueux, **caractérisé en ce que** la pression dans les branches veineuse et artérielle du circuit extracorporel est détectée dans des mesures consécutives, dans lequel la somme et la différence des pressions veineuse et artérielle dans le circuit extracorporel sont déterminées, et **en ce qu'**à partir de la somme et de la différence des pressions veineuse et artérielle, des valeurs caractéristiques pour l'état de l'accès vasculaire sont calculées.

2. Procédé selon la revendication 1, **caractérisé en ce que** pour le calcul d'une première valeur caractéristique pour l'état de l'accès vasculaire, la différence entre la somme des pressions veineuse et artérielle d'une mesure suivante et la somme des pressions veineuse et artérielle d'une mesure précédente est déterminée, et ces différences de pression de mesures consécutives sont additionnées, et pour le calcul d'une deuxième valeur caractéristique pour l'état de l'accès vasculaire, la différence entre la différence des pressions veineuse et artérielle d'une mesure suivante et la différence des pressions veineuse et artérielle d'une mesure précédente est déterminée et ces différences de pression de mesures consécutives sont additionnées en continu.

3. Procédé selon la revendication 2, **caractérisé en ce que** l'on vérifie si les première et deuxième valeurs caractéristiques sont positives ou négatives, et **en ce que** l'on compare les première et deuxième valeurs caractéristiques à des première et deuxième valeurs de seuil, dans lequel il est conclu à un accès vasculaire défectueux quand à la fois les première et deuxième valeurs caractéristiques sont négatives et la première valeur caractéristique est inférieure à la première valeur de seuil et la deuxième valeur caractéristique est inférieure à la deuxième valeur de seuil.

**4.** Procédé selon l'une quelconque des revendications 1 à 3, **caractérisé en ce que** quand un accès vasculaire défectueux est constaté, une alarme est donnée.

**5.** Procédé selon l'une quelconque des revendications 1 à 4, **caractérisé en ce que** quand un accès vasculaire défectueux est constaté, le flux sanguin dans le circuit extracorporel est interrompu.

**6.** Dispositif pour le traitement extracorporel du sang, comprenant

■ une conduite de sang artériel (5) d'un circuit extracorporel du sang, qui est reliée par une extrémité à l'entrée du dispositif de traitement du sang (1) et par l'autre extrémité à une connexion artérielle (5a) pour un accès vasculaire,
■ une conduite de sang veineux (7) du circuit extracorporel du sang, qui est reliée par une extrémité à la sortie du dispositif de traitement du sang (1) et par l'autre extrémité à une connexion veineuse (7a) pour l'accès vasculaire, et
■ un dispositif (17) pour surveiller l'accès vasculaire avec un capteur de pression artérielle (18) surveillant la pression $P_{art.}$ dans la conduite de sang artériel et un capteur de pression veineuse (19) surveillant la pression $P_{ven.}$ dans la conduite de sang veineux,

dans lequel

■ le dispositif pour surveiller l'accès vasculaire présente :

■ une unité de calcul (22) pour calculer des valeurs $W_1$, $W_2$ caractéristiques pour l'état de l'accès vasculaire à partir de la pression dans les branches artérielle et veineuse du circuit extracorporel, et
■ une unité d'évaluation (25) pour évaluer les valeurs caractéristiques afin de reconnaître un accès veineux défectueux,

**caractérisé en ce que** l'unité de calcul (22) présente des moyens pour calculer la somme et la différence des pressions veineuse et artérielle dans le circuit extracorporel, détectées dans des mesures consécutives.

**7.** Dispositif selon la revendication 6, **caractérisé en ce que** le dispositif (17) pour surveiller l'accès vasculaire présente des moyens (23) pour mémoriser les sommes et différences déterminées dans des mesures consécutives des pressions veineuse et artérielle, et **en ce que** l'unité de calcul (22) pour calculer une première valeur $W_1$ caractéristique pour l'état de l'accès vasculaire présente des moyens pour former la différence entre la somme des pressions veineuse et artérielle d'une mesure suivante et la somme des pressions veineuse et artérielle d'une mesure précédente et des moyens pour additionner ces différences de pression de mesures consécutives et pour calculer une deuxième valeur $W_2$ caractéristique pour l'état de l'accès vasculaire pour former la différence entre la différence des pressions veineuse et artérielle d'une mesure suivante et la différence des pressions veineuse et artérielle d'une mesure précédente et des moyens pour additionner ces différences de pression de mesures consécutives.

**8.** Dispositif selon la revendication 7, **caractérisé en ce que** l'unité d'évaluation (25) présente des moyens pour déterminer le signe des première et deuxième valeurs caractéristiques $W_1$, $W_2$ et des moyens pour comparer la première valeur caractéristique à une première valeur de seuille $M_1$ et des moyens pour comparer la deuxième valeur caractéristique $W_2$ à une deuxième valeur de seuil $M_2$.

**9.** Dispositif selon l'une quelconque des revendications 6 à 8, **caractérisé en ce qu'**un indicateur d'alarme (28) est prévu qui déclenche une alarme quand un accès vasculaire défectueux est constaté.

**10.** Dispositif selon l'une quelconque des revendications 6 à 9, **caractérisé en ce que** des moyens (16, 28) sont prévus pour interrompre le flux sanguin dans le circuit extracorporel du sang quand un accès vasculaire défectueux est constaté.

| Ursache | $P_{art.}$ | $P_{ven.}$ | $P_S$ | $\Delta P$ | $\sum_N d(P_s)$ | $\sum_N d(\Delta P)$ | Bemerkung |
|---|---|---|---|---|---|---|---|
| 1. Blutdruckabfall des Patienten | ↓ | ↓ | ↓ | → | - | 0 | |
| 2. Blutdruckanstieg des Patienten | ↑ | ↑ | ↑ | → | + | 0 | |
| 3. Viskositätserhöhung des Blutes | ↓ | ↑ | ↓ | ↑ | - | + | |
| 4. Viskositätserniedrigung des Blutes | ↑ | ↓ | ↑ | ↓ | + | - | |
| 5. Erhöhung von $Q_B$ | ↓ | ↑ | ↓ | ↑ | - | + | Abb. 4 |
| 6. Erniedrigung von $Q_B$ | ↑ | ↓ | ↑ | ↓ | + | - | Abb. 4 |
| 7. Lageänderung des Patienten nach oben | ↑ | ↑ | ↑ | → | + | 0 | |
| 8. Lageänderung des Patienten nach unten | ↓ | ↓ | ↓ | → | - | 0 | Abb. 5 |
| 9. Blutleck im arteriellen Schlauchsystem | ↑ | → | ↑ | ↑ | + | + | Durch vorhandene Schutzsysteme gesichert |
| 10. Blutleck im venösen Schlauchsystem | → | ↓ | ↓ | ↓ | - | - | Abb. 6; Unzureichend gesichert ! |
| 11. Herausrutschen der arteriellen Kanüle | ↑ | → | ↑ | ↑ | + | + | Durch vorhandene Schutzsysteme gesichert |
| 12. Herausrutschen der venösen Kanüle | ↓ | ↓ | ↓ | ↓ | - | - | Abb. 6; Unzureichend gesichert ! |

Einflüsse, die zur Änderung der Drucke im extrakorporalen Kreislauf beitragen.

↑ : Anstieg

↓ : Abfall

→ : Keine Änderung

Figur 1

Figur 2

EP 1 584 339 B1

Figur 3

Figur 4

Figur 5

Figur 6

Figur 7

16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9710013 A